# EUROPEAN PATENT SPECIFICATION

(11) **EP 4 048 829 B1**
(45) Date of publication and mention of the grant of the patent: **10.06.2026**
(21) Application number: 20879694.6
(22) Date of filing: 23.10.2020
(51) Int. Cl.: C40B 40/08, A61K 31/70, A61K 38/45, A61P 43/00, C12Q 1/68, C12Q 1/6806, C12Q 1/6869, C12Q 1/6883

(54) **SAMPLE PREPARATION AND SEQUENCING ANALYSIS FOR REPEAT EXPANSION DISORDERS AND SHORT READ DEFICIENT TARGETS**
PROBENVORBEREITUNG UND SEQUENZIERUNGSANALYSE FÜR REPEAT-EXPANSIONSSTÖRUNGEN UND SHORT-READ-DEFIZIENTE TARGETS
PRÉPARATION D'ÉCHANTILLONS ET ANALYSE PAR SÉQUENÇAGE DESTINÉES À DES TROUBLES DUS À L'EXPANSION DE RÉPÉTITIONS ET À DES CIBLES DÉFICIENTES EN SÉQUENCES LUES COURTES

(30) Priority: 23.10.2019 US 201962924922 P
(43) Date of publication of application: 31.08.2022
(73) Proprietor: Jumpcode Genomics, Inc., San Diego, CA 92121 (US)
(72) Inventor: BROWN, Keith, San Diego, California 92121 (US)
(74) Representative: J A Kemp LLP
(86) International application number: PCT/US2020/057163
(87) International publication number: WO 2021/081403

(56) References cited:
- WO-A1-2016/028887
- WO-A1-2016/028887
- WO-A1-2019/030306
- US-A1- 2014 134 610
- US-A1- 2014 357 523
- US-A1- 2015 011 403
- GILPATRICK TIMOTHY ET AL: "Targeted Nanopore Sequencing with Cas9 for studies of methylation, structural variants, and mutations", BIORXIV, 4 June 2019 (2019-06-04), XP055816504, Retrieved from the Internet <URL:https://www.biorxiv.org/content/10.1101/604173v2.full.pdf> [retrieved on 20210622], DOI: 10.1101/604173

## Description

### CROSS REFERNCE

This application claims the benefit of U.S. Provisional Application No. 62/924,922, filed on October 23, 2019.

### BACKGROUND

The disclosure herein relates to the field of molecular biology, such as methods and compositions for preparation and analysis of nucleic acids. Specifically, the disclosure relates to methods and compositions for sequence analysis, classifying multiple aspects of a repeat expansion disorder in a single sequencing assay, and for genotyping a target nucleic acid sequence

Repeat expansion disorders like Fragile X or Huntington disease are some of the more common genetic disorders. Fragile X being most common for Mendelian testing. Current molecular diagnostic testing is cumbersome, takes too long and requires multiple different technologies. Improved methods of sample preparation and sequencing analysis are needed for classifying repeat expansion disorders and short read deficient targets.

WO2016028887A1 discloses targeted enrichment of repeat expansion loci such as FMR1 or C9orf72 by Cas9 cleavage and adapter ligation, followed by long-read sequencing to determine repeat size, mosaicism and methylation.

Gilpatrick, T. et al., Targeted Nanopore Sequencing with Cas9 for studies of methylation, structural variants, and mutations, bioRxiv, DOI:10.1101/604173, describes Cas9-based enrichment strategies for nanopore sequencing, including evaluation of tandem repeats and DNA methylation.

US2014134610A1 relates to enrichment of repetitive regions such as the CGG repeats in FMR1 using restriction digestion and ligation of stem-loop adapters, enabling detection of allelic differences, mosaicism and methylation.

WO2019030306A1 discloses nucleic acid enrichment using Cas9 cleavage and hairpin adapter ligation for subsequent sequencing, including bisulfite conversion for epigenetic analysis.

US2014357523A1 describes targeted sequencing using a Cas9-transposase fusion protein to integrate sequencing adapters at specific genomic sites for rapid library preparation.

### SUMMARY

Described herein, in accordance with some embodiments, are methods for classifying multiple aspects of a repeat expansion disorder in a single sequencing assay. Some embodiments include providing target nucleic acids from a subject, each target nucleic acid comprising a repeat sequence, a first flanking region upstream of the repeat sequence, and a second flanking region downstream of the repeat sequence. Some embodiments include cleaving, with an enzyme, the first and second flanking regions to produce cleaved target nucleic acids each comprising the repeat sequence, a first end upstream of the repeat sequence, and a second end downstream of the repeat sequence. Some embodiments include connecting a first adapter nucleic acid to the first end, and a second adapter nucleic acid to the second end of each cleaved target nucleic acid to produce target nucleic acid products. Some embodiments include sequencing the target nucleic acid products. Some embodiments include identifying, based on the sequenced target nucleic acid products, a number of repeats in the repeat sequence of each target nucleic acid. In some embodiments, the target nucleic acids each comprise DNA such as genomic DNA. In some embodiments, the target nucleic acids each comprise RNA such as mRNA. Some embodiments include converting one or more non-methylated cytosines (C) on the target nucleic acids, cleaved target nucleic acids, or target nucleic acid products, to uracils (U) prior to sequencing the target nucleic acid products. In some embodiments, converting the non-methylated cytosines to uracils comprises treating the target nucleic acids, cleaved target nucleic acids, or target nucleic acid products, with a bisulfite. In some embodiments, a 5-methylcytosine status is identified for the target nucleic acids based on In some embodiments, the enzyme comprises a Cas9 enzyme. In some embodiments, cleaving, with an enzyme, the first and second flanking regions comprises using one or more guide nucleic acids to target the enzyme to the first and second flanking regions. In some embodiments, using one or more guide nucleic acids to target the enzyme to the first and second flanking regions comprises using 1, 2, 3, or 4, or more guide nucleic acid sequences. In some embodiments, using one or more guide nucleic acids to target the enzyme to the first and second flanking regions comprises using 4 guide nucleic acid sequences. In some embodiments, each guide nucleic acid sequence targets the enzyme to a separate target site of the first or second flanking region. In some embodiments, the first and second ends of the cleaved target nucleic acids are blunt ends. In some embodiments, the first and second ends of the cleaved target nucleic acids are sticky ends each comprising an overhang such as a 5' overhang or a 3' overhang. In some embodiments, the adapter nucleic acids comprise hairpin adapters. In some embodiments, the sequencing comprises multipass sequencing. In some embodiments, the adapter nucleic acids comprise sticky ends each comprising an overhang such as a 5' overhang or a 3' overhang. In some embodiments, connecting a first adapter nucleic acid to the first end, and a second adapter nucleic acid to the second end of each cleaved target nucleic acid comprises ligating the first and second adapter nucleic acids to the first and second ends of the cleaved target nucleic acids. In some embodiments, the enzyme comprises a transposase. In some embodiments, connecting a first adapter nucleic acid to the first end, and a second adapter nucleic acid to the second end of each cleaved target nucleic acid comprises using the transposase to connect ligating the first and second adapter nucleic acids to the first and second ends of the cleaved target nucleic acids. In some embodiments, the transposase is connected or tethered to a deactivated Cas9 enzyme that guides the transposase to the first and/or second flanking region. In some embodiments, one or more of the adapter nucleic acids each comprise a T7 promoter. In some embodiments, the adapter nucleic acids each comprise a sequencer flow cell binding sequence or primer initiation site. In some embodiments, the first and second adapter nucleic acids each comprise a unique molecule index (UMI) sequence. In some embodiments, identifying, based on the sequenced target nucleic acid products, a number of repeats in the repeat sequence of each target nucleic acid comprises identifying the number of repeats in the repeat sequence of each target nucleic acid using the UMI sequences to identify a separate sequence for each nucleic acid product. Some embodiments include digesting or degrading nucleic acids not comprising the target nucleic acid products. In some embodiments, the digestion is performed using an exonuclease. In some embodiments, the first and/or second adapter nucleic acids protect the target nucleic acid products from the digestion or degradation. In some embodiments, the first and/or second adapter nucleic acids comprise a phosphorothioate bond. In some embodiments, the repeat sequence comprises a Fragile X mental retardation 1 (FMR1) gene sequence. In some embodiments, the repeats each comprise a trinucleotide repeat. In some embodiments, the trinucleotide repeat comprises a CGG repeat. In some embodiments, the number of repeats in the repeat sequence of each target nucleic acid is 1-50, 50-100, 100-150, 150-200, or over 200. Some embodiments include identifying whether the subject has a genetic disorder based on the number of repeats in the repeat sequence of each target nucleic acid. In some embodiments, the genetic disorder is a repeat expansion disorder such as Fragile X syndrome, Huntington disease, amyotrophic lateral sclerosis, or spinocerebellar ataxia type 10. In some embodiments, the genetic disorder is Fragile X syndrome, and the subject is identified as having Fragile X syndrome when or if the number of repeats in the repeat sequence of a target nucleic acid is at least 200.

Described herein, in accordance with some embodiments, are methods for genotyping a target nucleic acid sequence. Some embodiments include providing a target nucleic acid from a subject, comprising the target sequence, a first flanking region upstream of the target sequence, and a second flanking region downstream of the target sequence. Some embodiments include cleaving, with an enzyme, the first and second flanking regions to produce a cleaved target nucleic acid comprising the target sequence, a first end upstream of the target sequence, and a second end downstream of the target sequence. Some embodiments include connecting a first adapter nucleic acid to the first end, and a second adapter nucleic acid to the second end of the cleaved target nucleic acid to produce a target nucleic acid product. Some embodiments include sequencing the target nucleic acid product. Some embodiments include identifying, based on the sequenced target nucleic acid product, a genotype of the target nucleic acid. In some embodiments, the target nucleic acid comprises DNA such as genomic DNA. In some embodiments, the target nucleic acid comprises RNA such as mRNA. In some embodiments, the enzyme comprises a Cas9 enzyme. In some embodiments, cleaving, with an enzyme, the first and second flanking regions comprises using one or more guide nucleic acids to target the enzyme to the first and second flanking regions. In some embodiments, using one or more guide nucleic acids to target the enzyme to the first and second flanking regions comprises using 1, 2, 3, or 4, or more guide nucleic acid sequences. In some embodiments, using one or more guide nucleic acids to target the enzyme to the first and second flanking regions comprises using 4 guide nucleic acid sequences. In some embodiments, each guide nucleic acid sequence targets the enzyme to a separate target site of the first or second flanking region. In some embodiments, the first and second ends of the cleaved target nucleic acid are blunt ends. In some embodiments, the first and second ends of the cleaved target nucleic acid are sticky ends each comprising an overhang such as a 5' overhang or a 3' overhang. In some embodiments, the adapter nucleic acids comprise hairpin adapters. In some embodiments, the sequencing comprises multipass sequencing. In some embodiments, the adapter nucleic acids comprise sticky ends each comprising an overhang such as a 5' overhang or a 3' overhang. In some embodiments, connecting a first adapter nucleic acid to the first end, and a second adapter nucleic acid to the second end of the cleaved target nucleic acid comprises ligating the first and second adapter nucleic acids to the first and second ends of the cleaved target nucleic acid. In some embodiments, the enzyme comprises a transposase. In some embodiments, connecting a first adapter nucleic acid to the first end, and a second adapter nucleic acid to the second end of each cleaved target nucleic acid comprises using the transposase to connect ligating the first and second adapter nucleic acids to the first and second ends of the cleaved target nucleic acid. In some embodiments, the transposase is connected or tethered to a deactivated Cas9 enzyme that guides the transposase to the first and/or second flanking region. In some embodiments, one or more of the adapter nucleic acids each comprise a T7 promoter. In some embodiments, the adapter nucleic acids each comprise a sequencer flow cell binding sequence or primer initiation site. In some embodiments, the first and second adapter nucleic acids each comprise a unique molecule index (UMI) sequence. Some embodiments include digesting or degrading nucleic acids not comprising the target nucleic acid product. In some embodiments, the digestion is performed using an exonuclease. In some embodiments, the first and/or second adapter nucleic acids protect the target nucleic acid product from the digestion or degradation. In some embodiments, the first and/or second adapter nucleic acids comprise a phosphorothioate bond. In some embodiments, the target nucleic acid sequence comprises a cytochrome P450 (CYP)-encoding sequence. In some embodiments, the CYP comprises CYP2D6. In some embodiments, target nucleic acid sequence comprises a sequence encoding HLA-A, HLA-B, HLA-B*1502, HLA-B*5701, CYPD6, CYP2C9, CYP2C19, CYP3A4, CYP3A5, ADRA2A, CYP1A2, CYP2B6, CYP4F2, VKORC1, COMT, DPYD, any of Factor II-Factor V Leiden, GRIK4, HTR2A, HTR2C, IFNL3, MTHFR, NAT2, OPRM1, SLCO1B1, SLC6A4, TPMT, UGT1A1, DRD3, D4D4, or TMPT.

### BRIEF DESCRIPTION OF THE DRAWINGS

**FIG. 1** depicts some embodiments where a CRISPR/CAS Nickase Creates Sticky ends flanking target region.
**FIG. 2** depicts an example of a target nucleic acid product.
**FIG. 3** depicts an example of some embodiments that include sample preparation with an RNA intermediate, and/or sequencing.
**FIG. 4A** depicts an embodiment that includes CRISPR induced insertion.
**FIG. 4B** depicts an embodiment that includes amplification of target DNA.
**FIG. 5** depicts an example where a standard dose is modified based on how well a person metabolizes a drug.
**FIG. 6A** is a chart showing a comparison of selected allele frequencies across world populations for CYP2D6.
**FIG. 6B** is a table showing some genes where a genotype is relevant to a disease or medicine.
**FIG. 7** is an image showing some information about Fragile X inheritance.
**FIG. 8** is a depiction of some aspects of Fragile X biology.
**FIG. 9** is a depiction of some aspects of Fragile X syndrome.
**FIG. 10** is a table showing some allele category definitions in common usage for Fragile X.

### DETAILED DESCRIPTION

Improved methods of sample preparation and sequencing analysis are provided herein for classifying a nucleotide repeat expansion disorders and short read deficient targets Fragile X is a trinucleotide expansion of CGG. It has been observed that normal individuals may have less than 50 repeats, whereas diseased individuals have > 200 repeats. Diagnosis in such cases should not only involve testing the repeat expansion itself, but also methylation status and mosaicism. The standard testing now is a southern blot for the length of the repeat, quantitative PCR for the mosaicism.

With oxford nanopore sequencing, a low cost, near sample result can be obtained by one assay. Targeted sequencing technology described in US Pat. Pub. No. 20190153528, can be used here. The targeted long read sequencing through an RNA intermediate can be used to diagnosing a nucleotide repeat expansion disorder, and with some additional steps.

Provided herein is a method for classifying multiple aspects of a nucleotide repeat expansion disorder in a single sequencing assay, the method involving sequence analysis of a genomic DNA region or a transcript generated thereof, further involving identifying methylated DNA in a quantitative and qualitative manner, overall facilitating determination of the multiplicity of a repeat sequence that could be the causal aspect for the nucleotide repeat expansion disease or disorder, and also the mosaicism of the repeats. The method comprises the following steps, providing target nucleic acids from a subject, each target nucleic acid comprising a repeat sequence, a first flanking region upstream of the repeat sequence, and a second flanking region downstream of the repeat sequence; cleaving, with an enzyme, the first and second flanking regions to produce cleaved target nucleic acids each comprising the repeat sequence, a first end upstream of the repeat sequence, and a second end downstream of the repeat sequence; connecting a first adapter nucleic acid to the first end, and a second adapter nucleic acid to the second end of each cleaved target nucleic acid to produce target nucleic acid products; sequencing the target nucleic acid products; and identifying, based on the sequenced target nucleic acid products, a number of repeats in the repeat sequence of each target nucleic acid.

In one aspect, provided herein is a method of genotyping. The method comprises the steps of providing a target nucleic acid from a subject, comprising the target sequence, a first flanking region upstream of the target sequence, and a second flanking region downstream of the target sequence; cleaving, with an enzyme, the first and second flanking regions to produce a cleaved target nucleic acid comprising the target sequence, a first end upstream of the target sequence, and a second end downstream of the target sequence; connecting a first adapter nucleic acid to the first end, and a second adapter nucleic acid to the second end of the cleaved target nucleic acid to produce a target nucleic acid product; and sequencing the target nucleic acid product; and identifying, based on the sequenced target nucleic acid product, a genotype of the target nucleic acid.

In some embodiments, the method is simple, fast and easy, and often less expensive than currently existing other methods known in the art.

In some embodiments, the method described herein comprises a step of cutting or nicking an upstream of a target nucleic acid using a nucleic acid guided nuclease e.g., CRISPR/Cas. Such cut/nicked nucleotide can be ligated with a nucleic acid comprising T7 promoter and/or a Unique Molecule Index (UMI) downstream of the T7 promoter. In some embodiments, Methyl-Cytosine (Methyl-C) in the target nucleic acid can be converted to uracil using bisulfite or any other chemical functioning similar to bisulfite. Such modified nucleic acid is sequenced. In some embodiments, the sequencing step can be performed in a micro sequencing device (e.g., nanopore device, Oxford MinION). In some embodiments, the method described herein can consist of a CRISPR cut upstream of the FLTR gene locus, ligation of a T7 promoter with a Unique Molecule Index (UMI) downstream of the T7 promoter, bisulfite or other conversion of Methyl-Cytosine (Methyl-C) to Uracil, RNA amplification, and sequencing on the sequencing device (e.g., micro device or handheld sequencing device (e.g., oxford minion)). The length of the repeat is detected through sequencing the entire molecule, the methylation status is determined by the C to U conversion and the mosaicism is determined by the Unique molecular identifier.

In some embodiments, the target nucleic acid comprises a repeat expansion sequence. The repeat expansion sequence includes triple nucleotide repeat expansion (e.g., CCG repeat, etc.). In some embodiments, the repeat expansion sequence includes at least 30, at least 40, at least 50, at least 60, at least 70, at least 80, at least 90, at least 100, at least 150, at least 200, at least 250, at least 300, at least 350, at least 400 repeats of triple nucleotide repeat. In some embodiment, the repeat expansion sequence is associated with an onset or development of Huntington's disease, fragile X syndrome, myotonic dystrophy, spinocerebellar ataxia, juvenile myoclonic epilepsy, and Friedreich's ataxia. In some embodiments, the target nucleic acid sequence comprises a cytochrome P450 (CYP)-encoding sequence. In some embodiments, the CYP comprises CYP2D6. In some embodiments, target nucleic acid sequence comprises a sequence encoding HLA-A, HLA-B, HLA-B*1502, HLA-B*5701, CYPD6, CYP2C9, CYP2C19, CYP3A4, CYP3A5, ADRA2A, CYP1A2, CYP2B6, CYP4F2, VKORC1, COMT, DPYD, any of Factor II-Factor V Leiden, GRIK4, HTR2A, HTR2C, IFNL3, MTHFR, NAT2, OPRM1, SLCO1B1, SLC6A4, TPMT, UGT1A1, DRD3, D4D4, or TMPT.

Alternatively and/or additionally, the method can comprise a step of cutting or nicking at loci flanking the target nucleic acid using nucleic acid guided nuclease e.g., CRISPR/Cas. Such cut/nicked nucleotide can be ligated with an adapter nucleic acid that is optionally including UMI. In some embodiments, Methyl-Cytosine (Methyl-C) in the target nucleic acid can be converted to uracil using bisulfite or any other chemical functioning similar to bisulfite. Such modified nucleic acid is sequenced. In some embodiments, the sequencing step can be performed in a micro sequencing device (e.g., nanopore device, Oxford MinION). In some embodiments, the method described herein would be to cut using CRISPR at loci flanking the FLTR gene, Ligate on adapters with UMIs, bisulfite conversion, then sequence. In some embodiments, the adapter is a hairpin adapter.

Alternatively and/or additionally, the method can comprise using a transposase tethered to a deactivated nuclease, such as CAS (e.g., CAS9) with guide nucleotide targeting the flanking region. In such embodiment, the deactivated CAS with guide nucleotide localizes the transposase at a targeted area by the guide nucleotide such that the transposase can act on the targeted area (e.g., inserting a nucleotide such as adapter sequence). In some embodiments, Methyl-Cytosine (Methyl-C) in the target nucleic acid can be converted to uracil using bisulfite or any other chemical functioning similar to bisulfite. Such modified nucleic acid is sequenced. In some embodiments, the sequencing step can be performed in a micro sequencing device (e.g., nanopore device, Oxford MinION). Another additional embodiment would be to use a combination comprising a transposase tethered to a deactivated CAS9 with guides targeting the flanking region. The guide RNA - CAS9 localizes the complex the right locations and the transposase inserts adapters at that site. This is followed by bisulfite conversion and sequencing. In some embodiments, the adapter is a hairpin adapter.

Alternatively and/or additionally, the method can comprise using a CAS derivative (e.g., CAS9 derivative) that only nicks the DNA on one strand to so generate sticky end of the nucleic acid. In some embodiments, the CAS derivative nicks at two strands in different location (e.g., nucleotide that is not complementary) such as flanking ends of the target nucleic acid (e.g., repeat expansion). The sticky end of the nucleic acid can be ligated with the adaptor sequence. In some embodiments, the adaptor sequence comprises a sticky end that is phosphorthioate modified, thus protected and/or stabilized. In some embodiments, the ligated nucleic acid can be treated with exonuclease. In some embodiments, Methyl-Cytosine (Methyl-C) in the target nucleic acid can be converted to uracil using bisulfite or any other chemical functioning similarly to bisulfite. Such modified nucleic acid is sequenced using UMI sequence(s). In some embodiments, the sequencing step can be performed in a micro sequencing device (e.g., nanopore device, Oxford MinION). In some embodiments, the adapter is a hairpin adapter. Another additional embodiment is to generate sticky end cuts by using a CAS9 derivative that only nicks the DNA on one strand, but do this at two locations (e.g., one location each in two strand, such as one in top strand and one in bottom strand) on both flanking ends of the repeat expansion. In some embodiments, the two locations are nucleotides that are not complementary with each other. In some embodiments, the two locations are nucleotides that are complementary with each other. Then adapters that are phosphorthioate protected ligates on the sticky end, then treated with exonuclease to digest the background. Then, conversion of methyl-cytosine or cytosine to uracil using bisulfite and then sequencing again using UMIs are performed to identify the mosaicism.

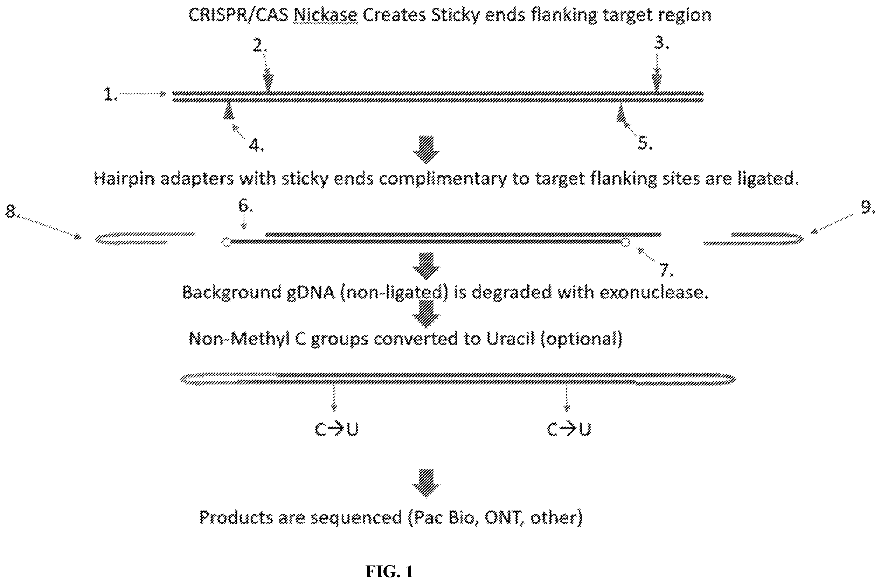

Some exemplary embodiments are shown in FIGs. 1-3. Some embodiments include the use of CRISPR-CAS nickase to make sticky ends flanking the target region. After this, two adapters are ligated to the resulting sticky end products. Some embodiments include a hairpin version or a protected double stranded adapter. The quadruple targeted of the CRISPR guides along with the hybridization and ligation of the sticky end hairpin adapters adds specificity. The background gDNA is then digested with exonuclease. The remaining products go through an optional bi-sulfite conversion (or other modification to convert unmethylated C bases to U) then the products are sequenced.

### Repeat Expansion Tests:

**Fragile X Market Size:** For testing a repeat sequence expansion related genetic disorder such as the Fragile X Syndrome, the market size appears to be $320M (Testing effected only). Fragile X Syndrome- (FMR1; CGG repeat) There appears to be a high prevalence of Fragile X Syndrome in all races/ethnic groups (1.6M effected) @ $200/ test, market size= $320M. Approximately 1 of 4000 males, and 1 of 6000 females are effected worldwide (source CDC); About 1 of 259 women, and 1 of 800 men are carriers = 15-30M for screening/ genetic counseling
- Specific indications for testing:
   ∘ Any male or female with autism or autistic-like characteristics (1:59 of 4M US births -> 68K autism/ year) >$13M/ year in US
   ∘ Any male or female with mental retardation (borderline to severe), developmental delay or learning disabilities of unknown cause.
   ∘ Any male or female with a relative who has fragile X syndrome or mental retardation of unknown cause.
   ∘ Anyone with a previous positive or equivocal result by the fragile X cytogenetic test.
   ∘ Confirmation of the presence of the FMR1 mutation is important.

**Other Repeat Expansion Tests:**
- Huntington's disease (HTT; CAG repeat): Prevalence estimated at 1/10,000 individuals in the US (30,000 affected individuals, 200,000 at-risk).
- Amyotrophic lateral sclerosis (ALS) and frontotemporal dementia (C9orf72; GGGGCC repeat): Prevalence of 1.7 in 100,000 individuals. ALS genetic tests costs $1600 to $5000.
- Spinocerebellar ataxia type 10 (SCA10) (ATXN10; variable ATTCT repeat).

### Definitions:

A partial list of definitions is as follows.

The term "repeat expansion", as used herein, refers to a region of a nucleic acid wherein a short sequence (as non-limiting examples, a trinucleotide, tetranucleotide or hexanucleotide) is repeated again and again. In some embodiments, the excessive number of repeats is in the coding segment of a gene. In some embodiments, an excessive number of repeats is associated with a particular disorder. In some embodiments, the repeat expansion is an expansion of a trinucleotide, tetranucleotide, or hexanucleotide repeat. In some embodiments, the repeat expansion is associated with a disorder selected from: neurological disorder, Huntington's disease, fragile X syndrome, fragile X-E syndrome, fragile X-associated tremor/ataxia syndrome, dystrophy, myotonic dystrophy, juvenile myoclonic epilepsy, ataxia, Friedreich's ataxia, spinocerebellar ataxia, atrophy, spino-bulbar muscular atrophy, Dentatorubropallidoluysian atrophy, ALS, frontotemporal lobar degeneration, frontotemporal dementia, and asthma. The terms "repeat disorder", "repeat expansion disorder" and the like, as used herein, refer to a pathological state which is associated with a repeat expansion, in which the number of adjacent trinucleotide repeats exceeds a number which is considered within the normal range, or below which is considered not to be associated with a particular disease. In some embodiments, a trinucleotide repeat disorder is a genetic disorder caused and/or associated with a trinucleotide repeat expansion, in which the number of adjacent trinucleotide repeats exceeds a number which is considered within the normal range, or below which is considered not to be associated with a particular disease.

"Amplified nucleic acid" or "amplified polynucleotide" is any nucleic acid or polynucleotide molecule whose amount has been increased at least two fold by any nucleic acid amplification or replication method performed *in vitro* as compared to its starting amount. For example, an amplified nucleic acid is obtained from a polymerase chain reaction (PCR) which can, in some instances, amplify DNA in an exponential manner (for example, amplification to 2ⁿ copies in n cycles). Amplified nucleic acid can also be obtained from a linear amplification.

"Amplification product" can refer to a product resulting from an amplification reaction such as a polymerase chain reaction.

An "amplicon" is a polynucleotide or nucleic acid that is the source and/or product of natural or artificial amplification or replication events.

The term "biological sample" or "sample" generally refers to a sample or part isolated from a biological entity. The biological sample may show the nature of the whole and examples include, without limitation, bodily fluids, dissociated tumor specimens, cultured cells, and any combination thereof. Biological samples can come from one or more individuals. One or more biological samples can come from the same individual. One non limiting example would be if one sample came from an individual's blood and a second sample came from an individual's tumor biopsy. Examples of biological samples can include but are not limited to, blood, serum, plasma, nasal swab or nasopharyngeal wash, saliva, urine, gastric fluid, spinal fluid, tears, stool, mucus, sweat, earwax, oil, glandular secretion, cerebral spinal fluid, tissue, semen, vaginal fluid, interstitial fluids, including interstitial fluids derived from tumor tissue, ocular fluids, spinal fluid, throat swab, breath, hair, finger nails, skin, biopsy, placental fluid, amniotic fluid, cord blood, emphatic fluids, cavity fluids, sputum, pus, microbiota, meconium, breast milk and/or other excretions. The samples may include nasopharyngeal wash. Examples of tissue samples of the subject may include but are not limited to, connective tissue, muscle tissue, nervous tissue, epithelial tissue, cartilage, cancerous or tumor sample, or bone. The sample may be provided from a human or animal. The sample may be provided from a mammal, including vertebrates, such as murines, simians, humans, farm animals, sport animals, or pets. The sample may be collected from a living or dead subject. The sample may be collected fresh from a subject or may have undergone some form of pre-processing, storage, or transport.

"Bodily fluid" generally can describe a fluid or secretion originating from the body of a subject. In some instances, bodily fluids are a mixture of more than one type of bodily fluid mixed together. Some non-limiting examples of bodily fluids are: blood, urine, bone marrow, spinal fluid, pleural fluid, lymphatic fluid, amniotic fluid, ascites, sputum, or a combination thereof.

"Complementary" or "complementarity" can refer to nucleic acid molecules that are related by base-pairing. Complementary nucleotides are, generally, A and T (or A and U), or C and G (or G and U). Two single stranded RNA or DNA molecules are said to be substantially complementary when the nucleotides of one strand, optimally aligned and with appropriate nucleotide insertions or deletions, pair with at least about 90% to about 95% complementarity, and more preferably from about 98% to about 100%) complementarity, and even more preferably with 100% complementarity. Alternatively, substantial complementarity exists when an RNA or DNA strand will hybridize under selective hybridization conditions to its complement. Selective hybridization conditions include, but are not limited to, stringent hybridization conditions. Hybridization temperatures are generally at least about 2° C to about 6° C lower than melting temperatures (Tₘ).

"Double-stranded" can refer to two polynucleotide strands that have annealed through complementary base-pairing.

"Known oligonucleotide sequence" or "known oligonucleotide" or "known sequence" can refer to a polynucleotide sequence that is known. A known oligonucleotide sequence can correspond to an oligonucleotide that has been designed, *e.g.,* a universal primer for next generation sequencing platforms (e.g., Illumina, 454), a probe, an adaptor, a tag, a primer, a molecular barcode sequence, an identifier. A known sequence can comprise part of a primer. A known oligonucleotide sequence may not actually be known by a particular user but is constructively known, for example, by being stored as data which may be accessible by a computer. A known sequence may also be a trade secret that is actually unknown or a secret to one or more users but may be known by the entity who has designed a particular component of the experiment, kit, apparatus or software that the user is using.

"Library" can refer to a collection of nucleic acids. A library can contain one or more target fragments. In some instances the target fragments are amplified nucleic acids. In other instances, the target fragments are nucleic acid that is not amplified. A library can contain nucleic acid that has one or more known oligonucleotide sequence(s) added to the 3' end, the 5' end or both the 3' and 5' end. The library may be prepared so that the fragments can contain a known oligonucleotide sequence that identifies the source of the library (*e.g.,* a molecular identification barcode identifying a patient or DNA source). In some instances, two or more libraries are pooled to create a library pool. Kits may be commercially available, such as the Illumina NEXTERA kit (Illumina, San Diego, CA).

The term "melting temperature" or "Tₘ" commonly refers to the temperature at which a population of double-stranded nucleic acid molecules becomes half dissociated into single strands. Equations for calculating the Tₘ of nucleic acids are well known in the art. One equation that gives a simple estimate of the Tₘ value is as follows: Tₘ=81.5+16.6(log 10[Na⁺])0.41(%[G+C])-675/n-1.0 m, when a nucleic acid is in aqueous solution having cation concentrations of 0.5 M or less, the (G+C) content is between 30% and 70%, n is the number of bases, and m is the percentage of base pair mismatches (see, *e.g.,* Sambrook J et al., Molecular Cloning, A Laboratory Manual, 3rd Ed., Cold Spring Harbor Laboratory Press (2001)). Other references can include more sophisticated computations, which take structural as well as sequence characteristics into account for the calculation of Tₘ.

"Nucleotide" can refer to a base-sugar-phosphate combination. Nucleotides are monomeric units of a nucleic acid sequence (*e.g.*, DNA and RNA). The term nucleotide includes naturally and non-naturally occurring ribonucleoside triphosphates ATP, TTP, UTP, CTG, GTP, and ITP, for example and deoxyribonucleoside triphosphates such as dATP, dCTP, dITP, dUTP, dGTP, dTTP, or derivatives thereof. Such derivatives can include, for example, [aS]dATP, 7-deaza-dGTP and 7-deaza-dATP, and, for example, nucleotide derivatives that confer nuclease resistance on the nucleic acid molecule containing them. The term nucleotide as used herein also refers to dideoxyribonucleoside triphosphates (ddNTPs) and their derivatives. Illustrative examples of dideoxyribonucleoside triphosphates include, ddATP, ddCTP, ddGTP, ddITP, ddUTP, ddTTP, for example. Other ddNTPs are contemplated and consistent with the disclosure herein, such as dd (2-6 diamino) purine.

"Polymerase" can refer to an enzyme that links individual nucleotides together into a strand, using another strand as a template.

"Polymerase chain reaction" or "PCR" can refer to a technique for replicating a specific piece of selected DNA *in vitro,* even in the presence of excess non-specific DNA. Primers are added to the selected DNA, where the primers initiate the copying of the selected DNA using nucleotides and, typically, Taq polymerase or the like. By cycling the temperature, the selected DNA is repetitively denatured and copied. A single copy of the selected DNA, even if mixed in with other, random DNA, is amplified to obtain thousands, millions, or billions of replicates. The polymerase chain reaction is used to detect and measure very small amounts of DNA and to create customized pieces of DNA.

The terms "polynucleotides" and "oligonucleotides" may include but is not limited to various DNA, RNA molecules, derivatives or combination thereof. These may include species such as dNTPs, ddNTPs, 2-methyl NTPs, DNA, RNA, peptide nucleic acids, cDNA, dsDNA, ssDNA, plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA. "Oligonucleotides," generally, are polynucleotides of a length suitable for use as primers, generally about 6-50 bases but with exceptions, particularly longer, being not uncommon.

A "primer" generally refers to an oligonucleotide used to prime nucleotide extension, ligation and/or synthesis, such as in the synthesis step of the polymerase chain reaction or in the primer extension techniques used in certain sequencing reactions. A primer may also be used in hybridization techniques as a means to provide complementarity of a locus to a capture oligonucleotide for detection of a specific nucleic acid region.

"Primer extension product" or "extension product" used interchangeably herein generally refer to the product resulting from a primer extension reaction using a contiguous polynucleotide as a template, and a complementary or partially complementary primer to the contiguous sequence.

"Sequencing," "sequence determination," and the like generally refers to any and all biochemical methods that may be used to determine the order of nucleotide bases in a nucleic acid.

A "sequence" as used herein refers to a series of ordered nucleic acid bases that reflects the relative order of adjacent nucleic acid bases in a nucleic acid molecule, and that can readily be identified specifically though not necessarily uniquely with that nucleic acid molecule. Generally, though not in all cases, a sequence requires a plurality of nucleic acid bases, such as 5 or more bases, to be informative although this number may vary by context. Thus a restriction endonuclease may be referred to as having a 'sequence' that it identifies and specifically cleaves even if this sequence is only four bases. A sequence need not 'uniquely map' to a fragment of a sample. However, in most cases a sequence must contain sufficient information to be informative as to its molecular source.

A "subject" generally refers to an organism that is currently living or an organism that at one time was living or an entity with a genome that can replicate. The methods, kits, and/or compositions of the disclosure is applied to one or more single-celled or multi-cellular subjects, including but not limited to microorganisms such as bacterium and yeast; insects including but not limited to flies, beetles, and bees; plants including but not limited to corn, wheat, seaweed or algae; and animals including, but not limited to: humans; laboratory animals such as mice, rats, monkeys, and chimpanzees; domestic animals such as dogs and cats; agricultural animals such as cows, horses, pigs, sheep, goats; and wild animals such as pandas, lions, tigers, bears, leopards, elephants, zebras, giraffes, gorillas, dolphins, and whales. The methods of this disclosure can also be applied to germs or infectious agents, such as viruses or virus particles or one or more cells that have been infected by one or more viruses.

A "support" is solid, semisolid, a bead, a surface. The support is mobile in a solution or is immobile.

The term "unique identifier" may include but is not limited to a molecular bar code, or a percentage of a nucleic acid in a mix, such as dUTP.

A "primer" as used herein refers to an oligonucleotide that anneals to a template molecule and provides a 3' OH group from which template-directed nucleic acid synthesis can occur. Primers comprise unmodified deoxynucleic acids in many cases, but in some cases comprise alternate nucleic acids such as ribonucleic acids or modified nucleic acids such as 2' methyl ribonucleic acids.

As used herein, a nucleic acid is double-stranded if it comprises hydrogen-bonded base pairings. Not all bases in the molecule need to be base-paired for the molecule to be referred to as double-stranded.

As used in the specification and claims, the singular forms "a", "an" and "the" include plural references unless the context clearly dictates otherwise. For example, the term "a sample" includes a plurality of samples, including mixtures thereof.

The term "about" as used herein in reference to a number refers to that number plus or minus up to 10% of that number. The term used in reference to a range refers to a range having a lower limit as much as 10% below the stated lower limit, and an upper number up to 10% above the stated limit.

Nucleic acids from a sample are obtained. Source "samples" may be derived from single cells, blood, urine, CSF, saliva (etc), environmental samples from soil, water, air... or cell free nucleic acids. Cells may be lysed to obtain the nucleic acid. Proper isolation/purification and removal of contaminants or nucleases when appropriate.

The circular structure of sequencing template (or target nucleic acid product) enables, in some embodiments, consensus sequencing (multipass sequencing) for technologies like Pacific biosciences.

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The section headings used herein are for organizational purposes only and are not to be construed as limiting the subject matter described.

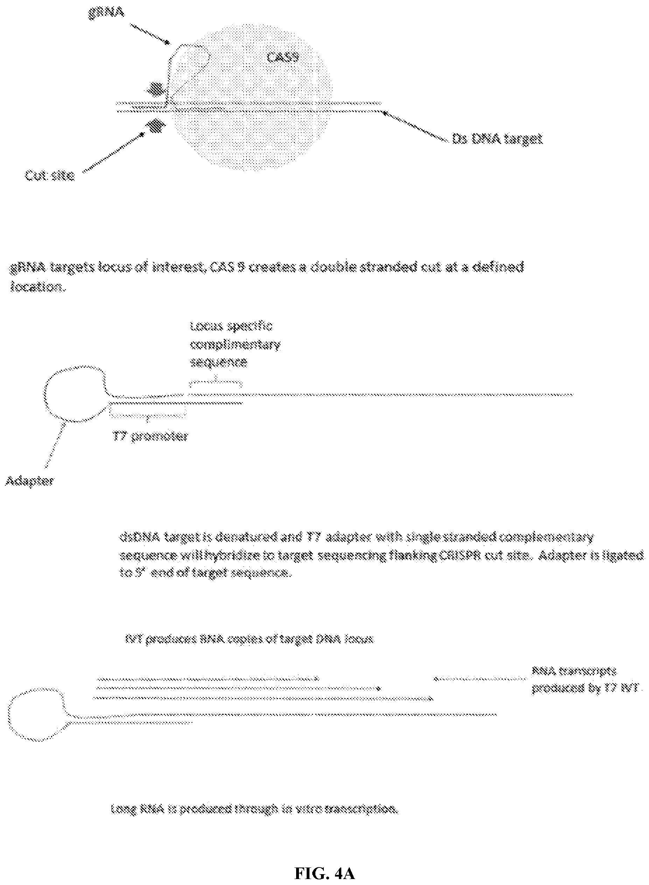

Some exemplary embodiments are shown in **FIG. 4A-4B****.** In some embodiments of a method for target specific RNA transcription of DNA sequences, CRISPR induced insertion of a hairpin nucleic acid with a double stranded T7 promoter to a target locus and subsequent linear amplification of the target locus. In some embodiments, *in vitro* transcription of target DNA generating amplified RNA copies of the target template for highly accurate short or long read sequencing.

### EXAMPLE 1. CYP2D6 PHARMACOGENOMIC TESTING

### Pharmacogenomics (PGX) Testing of CYP2D6

CYP2D6 is highly polymorphic, with over 100 known allelic variants and subvariants identified (www.pharmvar.org/gene/CYP2D6). It's involved in the metabolism of 25% of the most commonly prescribed drugs. Ultra-rapid and poor metabolizers may experience exaggerated/ potentially dangerous side effects or have reduced pharmacological effect. The CYP2D6 gene's highly polymorphic nature makes it very difficult to assay. In 2019, CAP proficiency testing showed that only 40- 50% 2D6 alleles were called correctly.

**Problem:** There is a pseudogene on same chromosome arm that makes short read sequencing mis-assign genotype calls.

**Current Methods:** Taqman/ primer extension/ microarrays all currently used; each approach has limitations.
- Arrays can assay the most variants/ but miss key alleles which need to be supplemented w individual Taqman assays.
- Taqman combined with array tape can test many alleles, but system is not easily replicated/ exported to multiple testing sites.
- Primer extension has multiplexing limitations

**Method:** Utilizing CRISPR, T7 promotors are inserted adjacent to the 4311-base pair 2D6 gene at 22q13.2. Full-length RNA synthesized for direct sequencing via PACB, Oxford Nanopore or ILMN. Universal approach can be used for development of additional molecular PGX assays.

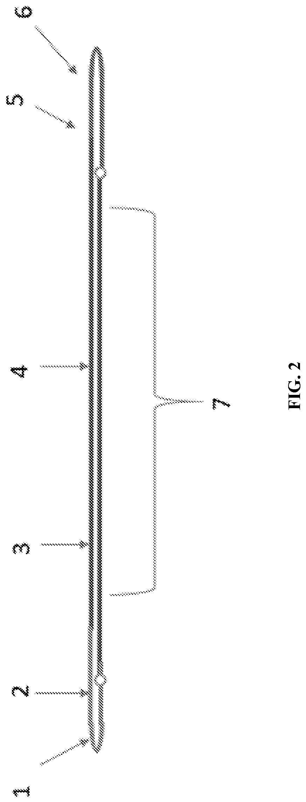

In general, the method comprises providing target nucleic acids from a subject. The subject may be suspected to have a CYP2D6 related disorder, and wherein the number 2D6 gene repeats are required to be assessed with precision. The each target nucleic acid, e.g., the 2D6 gene comprises the CYP2D6 repeat sequence as well as a first flanking region upstream of the repeat sequence, and a second flanking region downstream of the repeat sequence. The first and second flanking regions are cleaved with an enzyme to obtain the nucleic acid stretch containing the repeat sequence, the first end upstream of the repeat sequence, and the second end downstream of the repeat sequence. Next, a first adapter nucleic acid is annealed to the first end, and a second adapter nucleic acid to the second end of each cleaved target nucleic acid. This generates the nucleic acid product containing the target repeat sequence, the flanking regions and the adapters on either side. Next the nucleic acid product is sequenced and the number of repeats in the repeat sequence of each target nucleic acid is analyzed from the sequence reads. This general scheme is outlined in FIGs. 1 and 2. **Examples of components in** **FIG. 1****:** 1) Sample: double stranded Genomic DNA. 2) CRISPR/CAS Nickase guide RNA target site 1. 3) CRISPR/CAS Nickase guide RNA target site 2. 4) CRISPR/CAS Nickase guide RNA target site 3. 5) CRISPR/CAS Nickase guide RNA target site 4. 6) Sticky end created by CRISPR/CAS Nickase guide RNA target sites 1 and 2. 7) Sticky end created by CRISPR/CAS Nickase guide RNA target sites 3 and 4. 8) Hairpin adapter 1: Sequencing adapter sequence-unique molecule index-overhang complimentary to sticky end of target. 9) Hairpin adapter 2: Sequencing adapter sequence-unique molecule index-overhang complimentary to sticky end of target.

**Examples of components in** **FIG. 2****:** 1) Sequencer flow cell binding sequence/primer initiation site. 2) First Unique molecule Index: enables, in some embodiments, detection of mosaicism. 3) Example location of C→U conversion of unmethylated cytosine. 4) Example base location of methylated C residue (not converted to U). 5) Unique Molecule Index 2 for mosaicism. 6) Second sequencer flow cell binding sequence/primer initiation site (optional depending on sequencer platform). 7) Target sequence for genotyping and repeat expansion length.

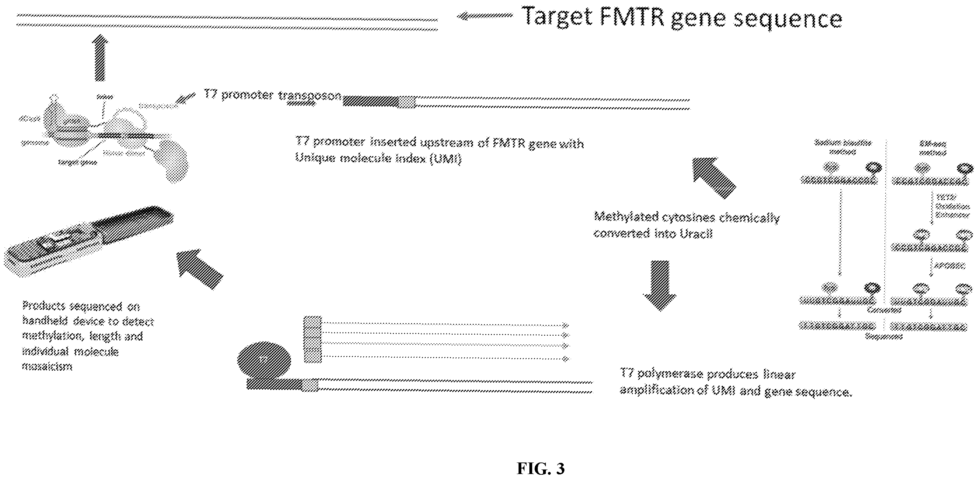

**FIG. 3** shows an exemplary workflow for determining the number of sequence repeats in FMTR gene (for example), in which genomic DNA comprising the repeat sequence is obtained, and a T7 promoter is introduced upstream of the repeats to allow linear amplification. Sequencing techniques known in the art allows to determine the number of repeats, the methylation status the length of the repeat stretch, and mosaicism.

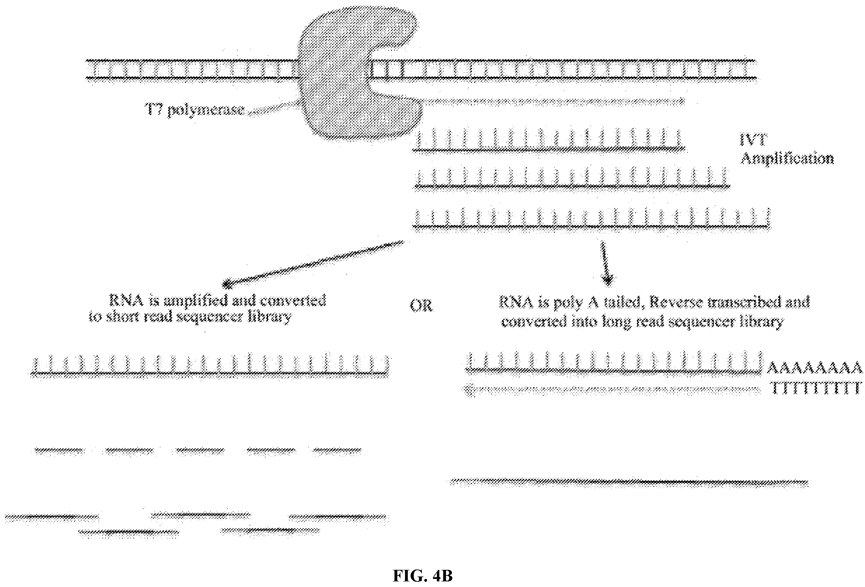

**FIGs 4A-4B** exemplifies insertion of a sequence with T7 promoter into a double-stranded cut close to the target repeat sequence and upstream using CRISPR-Cas9 system, followed by in vitro transcription as exemplified in **FIG. 4B****.**

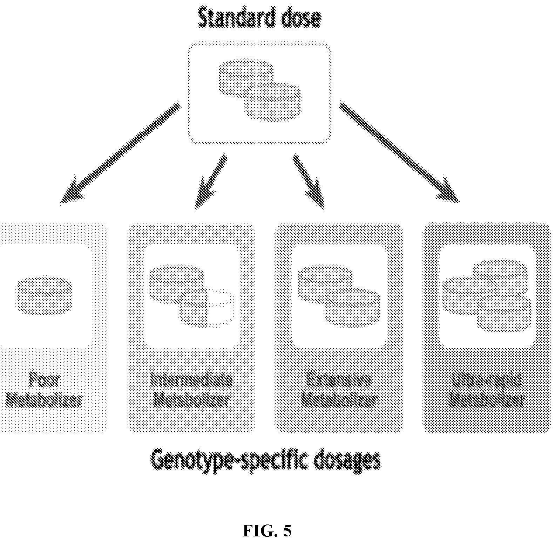

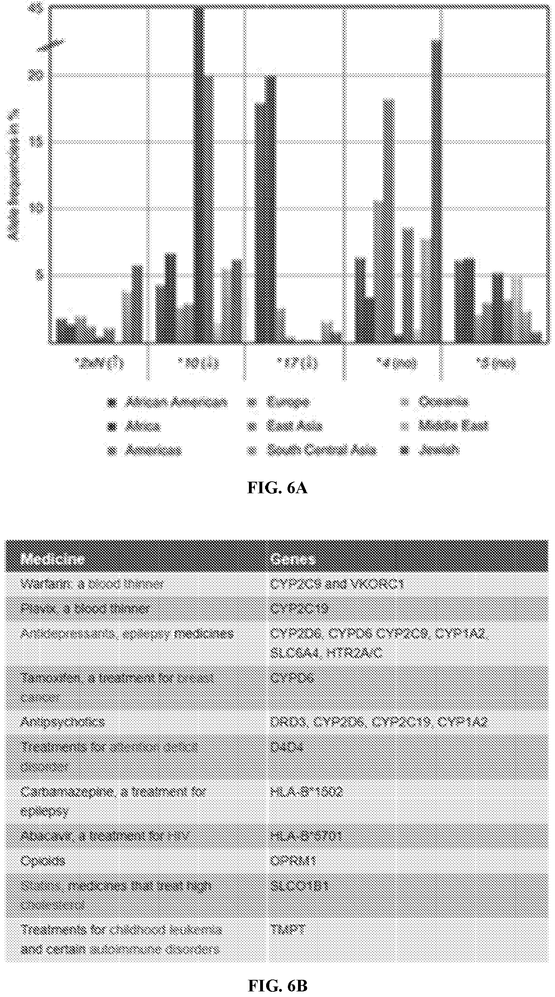

CYP2D6 metabolizes 25% of the most commonly prescribed drugs, such as antidepressants, antipsychotics, antitussives, beta adrenergic blocking agents, anti-arrhythmics, antiemetics and opioid analgesics (such as codeine, hydrocodone, dihydrocodeine, oxycodone, and tramadol) and the common breast cancer drug, Tamoxifen. (FIG. 6B) Therefore with the fast and accurate method described above for the determination of the number of repeats in the CYP2D6, users (e.g., medical practitioners) can then correlate the subject's ability to metabolize the CYP2D6 dependent drugs, and can modify the doses of the drugs accordingly **(****FIG. 5****).**

Some embodiments include prediction of a CYP2D6 phenotype.

**FIG. 6A** shows a comparison of selected allele frequencies across world populations for CYP2D6. Allele frequencies differ considerably between populations. **FIG. 6B** shows some genes where a genotype is relevant to a disease or medicine.

### EXAMPLE 2. FRAGILE X SYNDROME/ REPEAT NUCLEOTIDE EXPANSION DISEASE TESTING

**Summary:** There are over 30 disorders associated with repeat expansions including Fragile X syndrome, Huntington's disease, myotonic dystrophy, amyotrophic lateral sclerosis/fronto-temporal dementia, and spinocerebellar ataxias. Any male or female with autism or autistic-like characteristics (1:59 of US births) is being tested.

**Current Assays:** PCR recently replaced Southern blots as most often used assay. However, with genomic regions of extreme GC content and repetitive sequences, it's difficult to achieve consistent amplification via PCR. Correct diagnosis and more informed prognosis relies on accurate mutant repeat size estimates. Also, interruptions of the repeat may modulate stability, disease heritability and disease phenotype. So, in addition to repeat length that PCR based assays provide, it's important to obtain complete and accurate nucleotide-level sequence resolution of these regions

**Method:** Utilizing CRISPR, T7 promotors are inserted adjacent to regions of repeat expansions. Full-length RNA synthesized for direct sequencing via PACB or Oxford Nanopore. This universal approach can be used for development of other molecular assays.

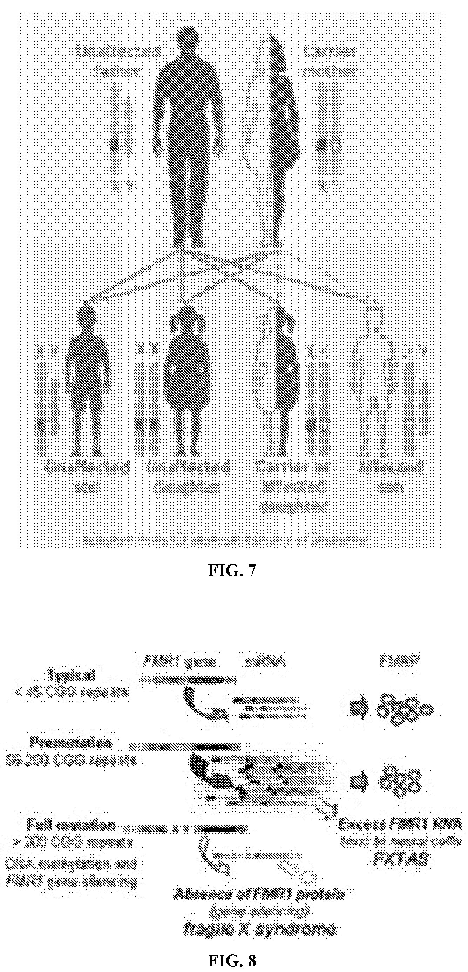

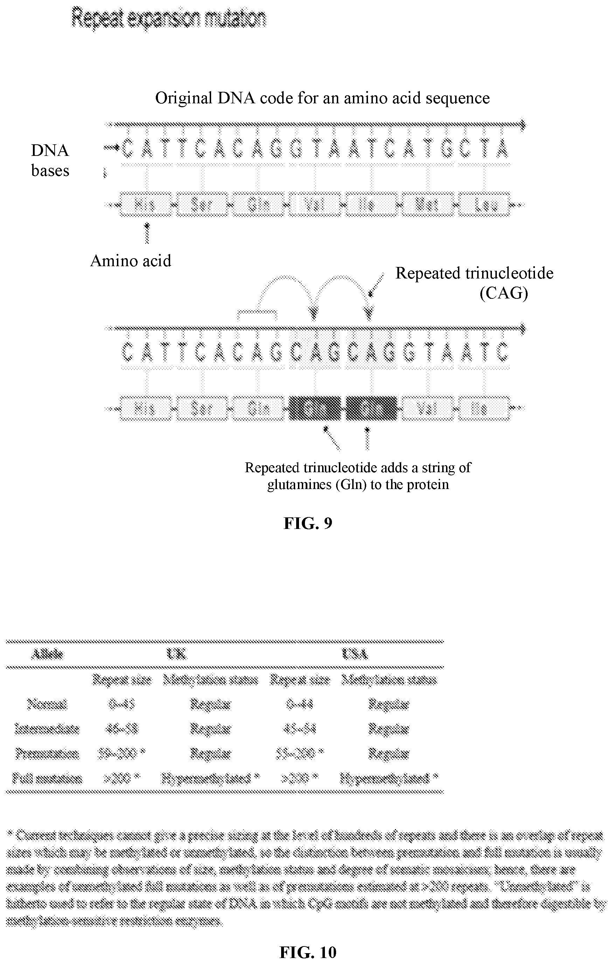

**FIGs. 7-10** provide information about Fragile X, Fragile X inheritance, Fragile X biology, and Fragile X allele categories. In some embodiments, the methods provided herein detect methylation status and repeat length and mosaicism in a single assay such as a single sequencing assay.

**Examples of components in** **FIG. 1****:** 1) Sample: double stranded Genomic DNA. 2) CRISPR/CAS Nickase guide RNA target site 1. 3) CRISPR/CAS Nickase guide RNA target site 2. 4) CRISPR/CAS Nickase guide RNA target site 3. 5) CRISPR/CAS Nickase guide RNA target site 4. 6) Sticky end created by CRISPR/CAS Nickase guide RNA target sites 1 and 2. 7) Sticky end created by CRISPR/CAS Nickase guide RNA target sites 3 and 4. 8) Hairpin adapter 1: Sequencing adapter sequence-unique molecule index-overhang complimentary to sticky end of target. 9) Hairpin adapter 2: Sequencing adapter sequence-unique molecule index-overhang complimentary to sticky end of target.

**Examples of components in** **FIG. 2****:** 1) Sequencer flow cell binding sequence/primer initiation site. 2) First Unique molecule Index: enables, in some embodiments, detection of mosaicism. 3) Example location of C→U conversion of unmethylated cytosine. 4) Example base location of methylated C residue (not converted to U). 5) Unique Molecule Index 2 for mosaicism. 6) Second sequencer flow cell binding sequence/primer initiation site (optional depending on sequencer platform). 7) Target sequence for genotyping and repeat expansion length.

The circular structure of sequencing template (or target nucleic acid product) enables, in some embodiments, consensus sequencing (multipass sequencing) for technologies like Pacific biosciences.

Some exemplary embodiments are shown in FIG. 4A-4B. In some embodiments of a method for target specific RNA transcription of DNA sequences, CRISPR induced insertion of a hairpin nucleic acid with a double stranded T7 promoter to a target locus and subsequent linear amplification of the target locus. In some embodiments, *in vitro* transcription of target DNA generating amplified RNA copies of the target template for highly accurate short or long read sequencing.

## Claims

1. A method for classifying a number of repeats and a mosaicism for a nucleotide repeat expansion disorder in a single sequencing assay, comprising:
providing target nucleic acids from a subject, each target nucleic acid comprising a repeat sequence, a first flanking region upstream of the repeat sequence, and a second flanking region downstream of the repeat sequence;
cleaving the first and second flanking regions with an enzyme to produce cleaved target nucleic acids each comprising the repeat sequence, a first end upstream of the repeat sequence, and a second end downstream of the repeat sequence;
connecting a first adapter nucleic acid to the first end, and a second adapter nucleic acid to the second end of each cleaved target nucleic acid to produce target nucleic acid products, wherein the first and second adapter nucleic acids each comprise a unique molecule index (UMI) sequence;
obtaining a sequence of the target nucleic acid products; and
identifying the number of repeats in the repeat sequence of each target nucleic acid based on the sequence of the target nucleic acid products using the UMI sequences to identify a separate sequence for each nucleic acid product from the subject, thereby determining the number of repeats and the mosaicism for the nucleotide repeat expansion disorder in the subject.

2. The method of claim 1, wherein the target nucleic acids comprise genomic DNA, RNA, or cDNA.

3. The method of claim 1 or claim 2, further comprising converting a non-methylated cytosine on the target nucleic acids, cleaved target nucleic acids, or target nucleic acid products, to uracils prior to sequencing the target nucleic acid products, optionally by treating the target nucleic acids, cleaved target nucleic acids, or target nucleic acid products, with a bisulfite, and optionally wherein a 5-methylcytosine status is identified for the target nucleic acids based on the number of cytosines and/or uracils in each of the sequenced target nucleic acid products.

4. The method of any of claims 1-3, wherein the enzyme comprises a Cas9 enzyme.

5. The method of any of claims 1-4, wherein cleaving comprises using one or more guide nucleic acids to target the enzyme to the first and second flanking regions.

6. The method of any of claims 1-5, wherein the adapter nucleic acids comprise hairpin adapters, optionally wherein obtaining a sequence comprises multipass sequencing.

7. The method of any of claims 1-6, wherein the first adapter nucleic acid comprises sticky ends each comprising an overhang.

8. The method of any of claims 1-7, wherein connecting the first adapter nucleic acid to the first end, and the second adapter nucleic acid to the second end of each cleaved target nucleic acid comprises ligating the first and second adapter nucleic acids to the first and second ends of the cleaved target nucleic acids.

9. The method of any of claims 1-8, wherein the enzyme comprises a transposase, and wherein the connecting the first adapter nucleic acid to the first end, and the second adapter nucleic acid to the second end of each cleaved target nucleic acid comprises using the transposase to connect ligating the first and second adapter nucleic acids to the first and second ends of the cleaved target nucleic acids, optionally wherein the transposase is connected or tethered to a deactivated Cas9 enzyme that guides the transposase to the first and/or second flanking region.

10. The method of any of claims 1-9, wherein one or more of the adapter nucleic acids each comprise a T7 promoter, a sequencer flow cell binding sequence, or a primer initiation site.

11. The method of any of claims 1-10, further comprising digesting or degrading nucleic acids not comprising the target nucleic acid products, optionally wherein the digestion is performed using an exonuclease, and optionally wherein the first and/or second adapter nucleic acids protect the target nucleic acid products from the digestion or degradation.

12. The method of any of claims 1-11, wherein the first and/or second adapter nucleic acids comprise a phosphorothioate bond.

13. The method of any of claims 1-12, wherein the repeats each comprise a trinucleotide repeat.

14. The method of any of claims 1-13, further comprising identifying whether the subject has a genetic disorder based on the number of repeats in the repeat sequence of each target nucleic acid, optionally wherein the genetic disorder is a nucleotide repeat expansion disorder such as Fragile X syndrome, Huntington disease, amyotrophic lateral sclerosis, or spinocerebellar ataxia type 10.

## Patentansprüche

1. Verfahren zum Klassifizieren einer Anzahl an Wiederholungen und eines Mosaizismus für eine Nukleotid-Repeatexpansionserkrankung in einem einzigen Sequenzierungsassay, umfassend:
Bereitstellen von Ziel-Nukleinsäuren von einem Subjekt, jede Ziel-Nukleinsäure umfassend eine Wiederholungssequenz, eine erste flankierende Region stromaufwärts von der Wiederholungssequenz und eine zweite flankierende Region stromabwärts von der Wiederholungssequenz umfasst;
Spalten der ersten und der zweiten flankierenden Region mit einem Enzym, um gespaltene Ziel-Nukleinsäuren zu erzeugen, jeweils umfassend die Wiederholungssequenz, ein erstes Ende stromaufwärts von der Wiederholungssequenz und ein zweites Ende stromabwärts von der Wiederholungssequenz;
Verbinden einer ersten Adapter-Nukleinsäure mit dem ersten Ende und einer zweiten Adapter-Nukleinsäure mit dem zweiten Ende von jeder gespaltenen Ziel-Nukleinsäure, um Ziel-Nukleinsäureprodukte zu erzeugen, wobei die erste und die zweite Adapter-Nukleinsäure jeweils eine Sequenz eindeutiger molekularer Identifikatoren (UMI) umfassen;
Erlangen einer Sequenz der Ziel-Nukleinsäureprodukte; und
Identifizieren der Anzahl an Wiederholungen in der Wiederholungssequenz jeder Ziel-Nukleinsäure basierend auf der Sequenz der Ziel-Nukleinsäureprodukte unter Verwendung der UMI-Sequenzen, um eine separate Sequenz für jedes Nukleinsäureprodukt von dem Subjekt zu identifizieren, wodurch die Anzahl an Wiederholungen und der Mosaizismus für die Nukleotid-Repeatexpansionserkrankung bei dem Subjekt bestimmt wird.

2. Verfahren nach Anspruch 1, wobei die Ziel-Nukleinsäuren genomische DNA, RNA oder cDNA umfassen.

3. Verfahren nach Anspruch 1 oder Anspruch 2, ferner umfassend ein Umwandeln eines nicht-methylierten Cytosins auf den Ziel-Nukleinsäuren, gespaltenen Ziel-Nukleinsäuren oder Ziel-Nukleinsäureprodukten in Uracile vor Sequenzieren der Ziel-Nukleinsäureprodukte, optional durch Behandeln der Ziel-Nukleinsäuren, gespaltenen Ziel-Nukleinsäuren oder Ziel-Nukleinsäureprodukte mit einem Bisulfit, und optional wobei ein 5-Methylcytosin-Status für die Ziel-Nukleinsäuren basierend auf der Anzahl an Cytosinen und/oder Uracilen in jedem der sequenzierten Ziel-Nukleinsäureprodukte identifiziert wird.

4. Verfahren nach einem der Ansprüche 1-3, wobei das Enzym ein Cas9-Enzym umfasst.

5. Verfahren nach einem der Ansprüche 1-4, wobei ein Spalten ein Verwenden einer oder mehrerer Guide-Nukleinsäuren umfasst, um das Enzym auf die erste und die zweite flankierende Region auszurichten.

6. Verfahren nach einem der Ansprüche 1-5, wobei die Adapter-Nukleinsäuren Haarnadeladapter umfassen, optional wobei ein Erlangen einer Sequenz Multipass-Sequenzierung umfasst.

7. Verfahren nach einem der Ansprüche 1-6, wobei die erste Adapter-Nukleinsäure klebrige Enden umfasst, jeweils umfassend einen Überhang.

8. Verfahren nach einem der Ansprüche 1-7, wobei ein Verbinden der ersten Adapter-Nukleinsäure mit dem ersten Ende und der zweiten Adapter-Nukleinsäure mit dem zweiten Ende jeder gespaltenen Ziel-Nukleinsäure ein Ligieren der ersten und der zweiten Adapter-Nukleinsäure mit dem ersten und dem zweiten Ende der gespaltenen Ziel-Nukleinsäuren umfasst.

9. Verfahren nach einem der Ansprüche 1-8, wobei das Enzym eine Transposase umfasst und wobei das Verbinden der ersten Adapter-Nukleinsäure mit dem ersten Ende und der zweiten Adapter-Nukleinsäure mit dem zweiten Ende jeder gespaltenen Ziel-Nukleinsäure ein Verwendung der Transposase umfasst, um ein Ligieren der ersten und der zweiten Adapter-Nukleinsäure mit dem ersten und dem zweiten Ende der gespaltenen Ziel-Nukleinsäuren zu verbinden, optional wobei die Transposase mit einem deaktivierten Cas9-Enzym verbunden oder daran gebunden ist, das die Transposase zu der ersten und/oder der zweiten flankierenden Region führt.

10. Verfahren nach einem der Ansprüche 1-9, wobei eine oder mehrere der Adapter-Nukleinsäuren jeweils einen T7-Promotor, eine Sequenzerfluss-Zellbindungssequenz oder eine Primer-Initiationsstelle umfassen.

11. Verfahren nach einem der Ansprüche 1-10, ferner umfassend ein Digerieren oder Abbauen von Nukleinsäuren, die nicht die Ziel-Nukleinsäureprodukte umfassen, optional wobei das Digerieren unter Verwendung einer Exonuklease durchgeführt wird und wobei die erste und/oder die zweite Adapter-Nukleinsäuren die Ziel-Nukleinsäureprodukte vor Digerieren oder Abbauen schützen.

12. Verfahren nach einem der Ansprüche 1-11, wobei die erste und/oder die zweite Adapter-Nukleinsäure eine Phosphorothioat-Bindung umfasst.

13. Verfahren nach einem der Ansprüche 1-12, wobei die Wiederholungen jeweils eine Trinukleotidwiederholung umfassen.

14. Verfahren nach einem der Ansprüche 1-13, ferner umfassend ein Identifizieren, ob das Subjekt eine genetische Erkrankung aufweist, basierend auf der Anzahl an Wiederholungen in der Wiederholungssequenz jeder Ziel-Nukleinsäure, wobei die genetische Erkrankung optional eine Nukleotid-Repeatexpansionserkrankung ist, wie beispielsweise fragiles X-Syndrom, Huntington-Krankheit, amyotrophe Lateralsklerose oder spinozerebelläre Ataxie Typ 10.

## Revendications

1. Procédé pour classifier un nombre de répétitions et un mosaïcisme pour un trouble d'expansion de répétitions de nucléotides dans un seul essai de séquençage, comprenant :
la fourniture d'acides nucléiques cibles provenant d'un sujet, chaque acide nucléique cible comprenant une séquence de répétitions, une première région flanquante en amont de la séquence de répétitions, et une seconde région flanquante en aval de la séquence de répétitions ;
le clivage des première et seconde régions flanquantes avec un enzyme pour produire des acides nucléiques cibles clivés, chacun comprenant la séquence de répétitions, une première extrémité en amont de la séquence de répétitions, et une seconde extrémité en aval de la séquence de répétitions ;
la jonction d'un premier acide nucléique adaptateur à la première extrémité, et d'un second acide nucléique adaptateur à la seconde extrémité de chaque acide nucléique cible clivé pour produire des produits acides nucléiques cibles, dans lequel les premier et second acides nucléiques adaptateurs comprennent chacun une séquence d'indice moléculaire unique (Unique Molecule Index, UMI) ;
l'obtention d'une séquence des produit acides nucléiques cibles ; et
l'identification du nombre de répétitions dans la séquence de répétitions de chaque acide nucléique cible sur la base de la séquence des produit acides nucléiques cibles en utilisant les séquences UMI pour identifier une séquence distincte pour chaque produit acide nucléique provenant du sujet, déterminant ainsi le nombre de répétitions et le mosaïcisme pour le trouble d'expansion de répétitions de nucléotides chez le sujet.

2. Procédé de la revendication 1, dans lequel les acides nucléiques cibles comprennent ADN, ARN, ou cADN génomique.

3. Procédé de la revendication 1 ou la revendication 2, comprenant en outre la conversion d'une cytosine non méthylée sur les acides nucléiques cibles, acides nucléiques cibles clivés, ou produits acides nucléiques cibles, en uraciles avant le séquençage des produit acides nucléiques cibles, facultativement en traitant les acides nucléiques cibles, acides nucléiques cibles clivés, ou produits acides nucléiques cibles, avec un bisulfite, et facultativement dans lequel un état 5-méthylcytosine est identifié pour les acides nucléiques cibles sur la base du nombre de cytosines et/ou d'uraciles dans chacun des produits acides nucléiques cibles séquencés.

4. Procédé de quelconques des revendications 1 à 3, dans lequel l'enzyme comprend une enzyme Cas9.

5. Procédé de quelconques des revendications 1 à 4, dans lequel le clivage comprend l'utilisation d'un ou de plusieurs acides nucléiques guides pour cibler l'enzyme vers les première et seconde région flanquantes.

6. Procédé de quelconques des revendications 1 à 5, dans lequel les acides nucléiques adaptateurs comprennent des adaptateurs en épingle à cheveux, facultativement dans lequel l'obtention d'une séquence comprend un séquençage multipasse.

7. Procédé de quelconques des revendications 1 à 6, dans lequel le premier acide nucléique adaptateur comprend des extrémités cohésives, chacune comprenant un dépassement.

8. Procédé de quelconques des revendications 1 à 7, dans lequel la jonction du premier acide nucléique adaptateur à la première extrémité, et du second acide nucléique adaptateur à la seconde extrémité de chaque acide nucléique cible clivé comprend la ligature des premier et second acides nucléiques adaptateurs aux première et seconde extrémités des acides nucléiques cibles clivés.

9. Procédé de quelconques des revendications 1 à 8, dans lequel l'enzyme comprend une transposase, et dans lequel la jonction du premier acide nucléique adaptateur à la première extrémité, et du second acide nucléique adaptateur à la seconde extrémité de chaque acide nucléique cible clivé comprend l'utilisation de la transposase pour joindre par ligature les premier et second acides nucléiques adaptateurs aux première et seconde extrémités des acides nucléiques cibles clivés, facultativement dans lequel la transposase est liée ou attachée à une enzyme Cas9 désactivée qui guide la transposase vers les première et/ou seconde régions flanquantes.

10. Procédé de quelconques des revendications 1 à 9, dans lequel un ou plusieurs des acides nucléiques adaptateurs comprennent chacun un promoteur T7, une séquence de liaison de cytométrie en flux avec séquenceur, ou un site d'initiation d'amorce.

11. Procédé de quelconques des revendications 1 à 10, comprenant en outre la digestion ou la dégradation d'acides nucléiques ne comprenant pas les produit acides nucléiques cibles, facultativement dans lequel la digestion est réalisée en utilisant une exonucléase, et facultativement dans lequel les premier et/ou second acides nucléiques adaptateurs protègent les produit acides nucléiques cibles de la digestion ou dégradation.

12. Procédé de quelconques des revendications 1 à 11, dans lequel les premier et/ou second acides nucléiques adaptateurs comprennent une liaison phosphorothioate bond.

13. Procédé de quelconques des revendications 1 à 12, dans lequel les répétitions comprennent chacune une répétition de trinucléotides.

14. Procédé de quelconques des revendications 1 à 13, comprenant en outre l'identification du fait que le sujet présente ou non un trouble génétique, sur la base du nombre de répétitions dans la séquence de répétitions de chaque acide nucléique cible, facultativement dans lequel le trouble génétique est un trouble d'expansion de répétitions de nucléotides, tel que le syndrome de l'X fragile, la maladie de Huntington, la sclérose latérale amyotrophique, ou l'ataxie spinocérébelleuse type 10.
